(19) Europäisches Patentamt
European Patent Office
Office européen des brevets

(11) EP 4 650 039 A1

(12) **EUROPEAN PATENT APPLICATION**
published in accordance with Art. 153(4) EPC

(43) Date of publication:
**19.11.2025 Bulletin 2025/47**

(21) Application number: **24741551.6**

(22) Date of filing: **11.01.2024**

(51) International Patent Classification (IPC):
***B01D 71/52*** (2006.01) ***A61M 1/18*** (2006.01)
***A61M 1/34*** (2006.01) ***A61M 1/36*** (2006.01)
***B01D 63/00*** (2006.01) ***B01D 65/02*** (2006.01)
***B01D 69/00*** (2006.01) ***B01D 71/44*** (2006.01)
***B01D 71/68*** (2006.01) ***B01D 71/80*** (2006.01)
***B01D 71/82*** (2006.01)

(52) Cooperative Patent Classification (CPC):
**B01D 65/022; A61M 1/16; A61M 1/34; A61M 1/36; B01D 63/00; B01D 63/02; B01D 65/02; B01D 69/00; B01D 71/44; B01D 71/441; B01D 71/52; B01D 71/68; B01D 71/76; B01D 71/80; B01D 71/82;** (Cont.)

(86) International application number:
**PCT/JP2024/000352**

(87) International publication number:
**WO 2024/150779 (18.07.2024 Gazette 2024/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.01.2023 JP 2023003901**

(71) Applicant: **Toyobo Co., Ltd.**
**Kita-ku**
**Osaka-shi, Osaka 530-0001 (JP)**

(72) Inventors:
- **KANAI, Taisei**
**Otsu-shi, Shiga 520-0292 (JP)**
- **KOYAMA, Shinya**
**Otsu-shi, Shiga 520-0292 (JP)**

(74) Representative: **Vossius & Partner**
**Patentanwälte Rechtsanwälte mbB**
**Siebertstrasse 3**
**81675 München (DE)**

(54) **SEMI-PERMEABLE MEMBRANE AND SEMI-PERMEABLE MEMBRANE MODULE**

(57) A semipermeable membrane comprises a sulfonated polyarylene ether copolymer and is sterilized with ethylene oxide gas. The sulfonated polyarylene ether copolymer includes a hydrophobic segment repeating unit represented by a formula (1) and a hydrophilic segment repeating unit represented by a formula (2) as copolymerization components.

FIG.1

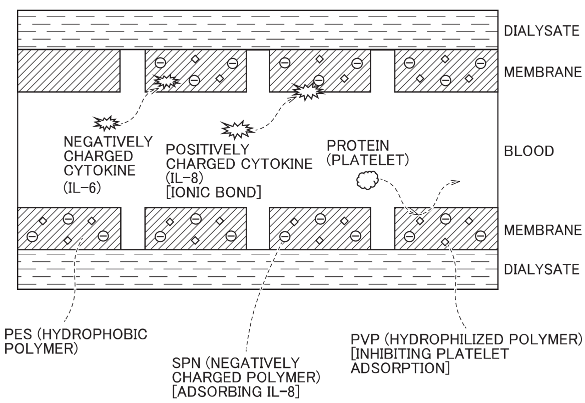


EP 4 650 039 A1

(52) Cooperative Patent Classification (CPC): (Cont.)
B01D 2323/2187

## Description

TECHNICAL FIELD

The present invention relates to a semipermeable membrane and a semipermeable membrane module.

BACKGROUND ART

Blood purification therapy based on membrane separation techniques such as hemodialysis, hemofiltration, and hemodiafiltration has been increasingly employed in recent years not only for patients with chronic renal failure but also for patients with acute renal failure, and also widely employed in critical care, ICU, and the like.

By the way, infection with COVID-19 can be accompanied by acute renal disorders with a sudden loss of kidney function and/or by a cytokine storm which is a severe immune reaction against viruses in which an excessive amount of proinflammatory mediators circulate in blood. Among patients with serious symptoms of COVID-19, 15 to 30% develop acute renal disorders and 67% develop organ dysfunction syndrome which is considered ascribable to an excessive amount of cytokines in circulation.

There is a therapy known as continuous renal replacement therapy (CRRT), which is for acute renal failure, organ dysfunction syndrome, and the like and which involves performing body fluid correction over a prolonged period of time in a slow manner (at a low circulation flow rate) as compared to the therapy conditions for ordinary hemodialysis and hemofiltration. CRRT is employed not only for replacing renal functions such as fluid removal, waste removal, and electrolyte balance correction but also for removing proinflammatory mediators, and, for instance, CRRT can be carried out as a single-round treatment at a blood flow rate from 50 to 150 mL/minute and a filtration rate of 5 to 50 mL/minute over 24 to 48 hours.

As a semipermeable membrane (a blood treatment apparatus) for CRRT, SepXiris (registered trademark) manufactured by Baxter Limited is known, for example. The semipermeable membrane used in SepXiris contains, as a component, a copolymer of acrylonitrile and sodium methallyl sulfonate. It is conceivable that this semipermeable membrane is mainly capable of adsorbing positively charged cytokines because its negatively charged sulfonate groups can form ionic bonds with amino groups of positively charged cytokines (see The Journal of Artificial Organs, vol. 43, No. 3, pp. 233-237, 2014 (NPL 1)).

Moreover, Japanese Patent No. 6817240 (Japanese Patent Laying-Open No. 2018-171431 (PTL 1)) discloses a hollow fiber membrane for blood purification that has hemocompatibility and cytokine adsorption capabilities. This hollow fiber membrane contains polymethyl methacrylate and sodium p-styrenesulfonate, and due to the negatively charged sulfonate groups, it is conceivable that the membrane is mainly capable of adsorbing positively charged cytokines.

Moreover, Japanese Patent Laying-Open No. 2001-70767 (PTL 2) discloses an ultrafiltration membrane for surface water treatment comprising a composition made of polyethersulfone, polyvinylpyrrolidone, and sulfonated polyethersulfone, which is, however, an ultrafiltration membrane for the purpose of water treatment and not accompanied by any mention regarding sterilization.

Furthermore, Japanese Patent No. 2728814 (Japanese Patent Laying-Open No. 5-064662 (PTL 3)) discloses sterilization treatment of a medical instrument comprising a separation membrane, with the use of ethylene oxide gas or radiation.

CITATION LIST

PATENT LITERATURE

PTL 1: Japanese Patent No. 6817240 (Japanese Patent Laying-Open No. 2018-171431)
PTL 2: Japanese Patent Laying-Open No. 2001-70767
PTL 3: Japanese Patent No. 2728814 (Japanese Patent Laying-Open No. 5-064662)

NON PATENT LITERATURE

NPL 1: The Journal of Artificial Organs, vol. 43, No. 3, pp. 233-237, 2014

SUMMARY OF INVENTION

TECHNICAL PROBLEM

The above-mentioned conventional semipermeable membrane for continuous renal replacement therapy is highly capable of adsorbing positively charged substances, but it is not very capable of adsorbing negatively charged substances, which is a drawback.

It should be noted that semipermeable membranes for medical use are required to be sterilized.

Hence, an object of the present invention is to provide a sterilized semipermeable membrane that is highly capable of adsorbing proinflammatory mediators (both negatively charged substances and positively charged substances).

SOLUTION TO PROBLEM

[1] A semipermeable membrane comprising:

a sulfonated polyarylene ether copolymer, wherein
the sulfonated polyarylene ether copolymer includes a hydrophobic segment repeating unit represented by a formula (1) below and a hydrophilic segment repeating unit represented by a formula (2) below as copolymerization components:

[Chem. 1]

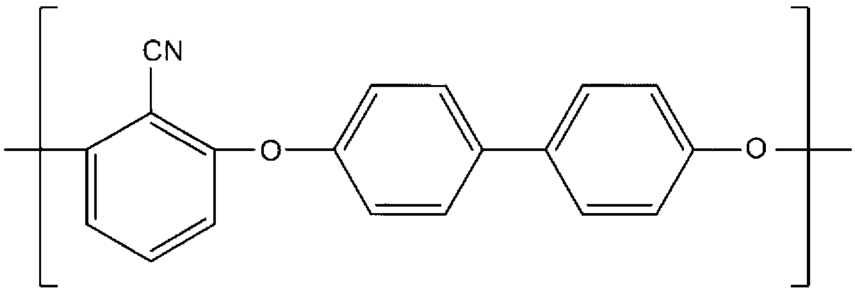

(1)

[Chem. 2]

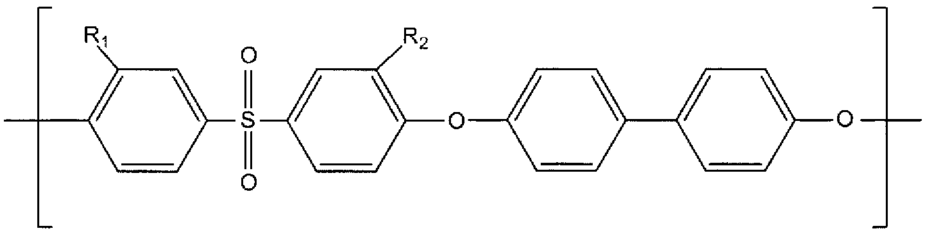

(2)

where a component ratio (in mole) of the formula (1) is from 0.4 to 0.7, a component ratio (in mole) of the formula (2) is from 0.3 to 0.6, a sum of the component ratio (in mole) of the formula (1) and the component ratio (in mole) of the formula (2) is 1.00, each of $R_1$ and $R_2$ represents "$-SO_3M$", and M represents a metallic element, and
the semipermeable membrane is sterilized with ethylene oxide gas.

[2] The semipermeable membrane according to [1], comprising polyethersulfone, the sulfonated polyarylene ether copolymer, and polyvinylpyrrolidone.

[3] The semipermeable membrane according to [1] or [2], for continuous renal replacement therapy.

[4] The semipermeable membrane according to any one of [1] to [3], having a structure that is not uniform in a thickness direction.

[5] A semipermeable membrane module comprising the semipermeable membrane according to any one of [1] to [4], wherein
the semipermeable membrane module is sterilized with ethylene oxide gas.

[6] The semipermeable membrane module according to [5], for continuous renal replacement therapy.

ADVANTAGEOUS EFFECTS OF INVENTION

The present invention makes it possible to provide a sterilized semipermeable membrane that is highly capable of adsorbing proinflammatory mediators (both negatively charged substances and positively charged substances).

BRIEF DESCRIPTION OF DRAWINGS

Fig. 1 is a schematic view for explaining a semipermeable membrane according to an embodiment.
Fig. 2 is a GPC chromatogram showing elution of degraded SPN after γ-ray sterilization.
Fig. 3 is a graph showing the relationship between the amount of elution of degraded SPN and UV absorbance.
Fig. 4 is a graph showing the relationship between γ-ray dose for sterilization and the amount of elution of degraded SPN.
Fig. 5 is an SEM photograph of a semipermeable membrane according to an embodiment.

DESCRIPTION OF EMBODIMENTS

In the following, a description will be given of embodiments of the present invention, but the scope of the present invention is not limited to these embodiments.

<Semipermeable Membrane>

A semipermeable membrane according to the present embodiment comprises a sulfonated polyarylene ether copolymer (a negatively charged polymer) and is sterilized with ethylene oxide gas.

Preferably, the semipermeable membrane according to the present embodiment comprises polyethersulfone (a hydrophobic polymer), a sulfonated polyarylene ether copolymer (a negatively charged polymer), and polyvinylpyrrolidone (a hydrophilic polymer).

Preferably, the semipermeable membrane according to the present embodiment is a semipermeable membrane for continuous renal replacement therapy, capable of removing proinflammatory mediators.

Proinflammatory mediators are biologically active substances (pain-producing substances, prostanoids, cytokines) that are released by leucocytes, mast cells, macrophages, and the like infiltrated in damaged tissue and inflammation sites. Examples of proinflammatory mediators include positively charged substances and negatively charged substances.

Examples of the positively charged substances include interleukin-8 (IL-8), interleukin-10 (IL-10), transforming growth factor β (TGF-β), monocyte chemotaxis promoting factor (MCP1), platelet-derived growth factor (PDGF), and the like.

Examples of the negatively charged substances include interleukin-6 (IL-6), interleukin-18 (IL-18), interleukin-1β (IL-1β), HMGB1 (High Mobility Group Box 1), tumor necrosis factor-α (TNF-α), and the like.

Referring to Fig. 1, it is conceivable that a positively charged substance (cytokine) such as IL-8 can form an ionic bond with a sulfonated polyarylene ether copolymer (SPN) which is a negatively charged polymer, and be adsorbed on the semipermeable membrane.

It is conceivable that polyvinylpyrrolidone which is a hydrophilicity-rendering agent (a hydrophilic polymer) can inhibit adsorption of proteins such as platelets onto the semipermeable membrane.

Although the action mechanism is unclear, the semipermeable membrane according to the present embodiment has enhanced capabilities of adsorbing negatively charged substances (cytokines) such as IL-6 as compared to conventional semipermeable membranes.

(Polyethersulfone)

The polyethersulfone is a compound that includes a structural unit represented by the following formula (3). The polyethersulfone is expected to act to adsorb proinflammatory mediators via hydrophobic interaction.

[Chem. 3]

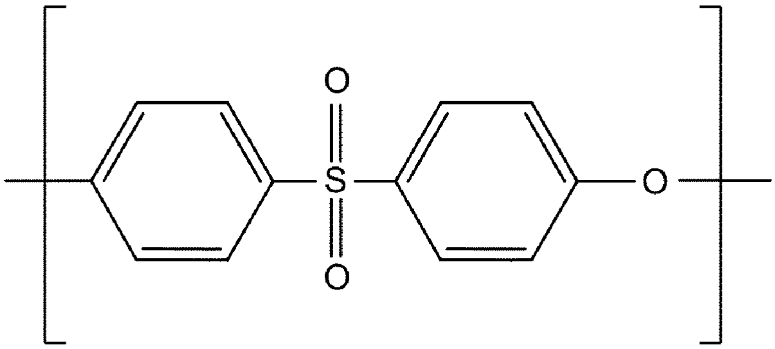

(3)

(Sulfonated Polyarylene Ether Copolymer)

The sulfonated polyarylene ether copolymer is a compound that includes a hydrophobic segment repeating unit represented by the following formula (1) and a hydrophilic segment repeating unit represented by the following formula (2).

[Chem. 4]

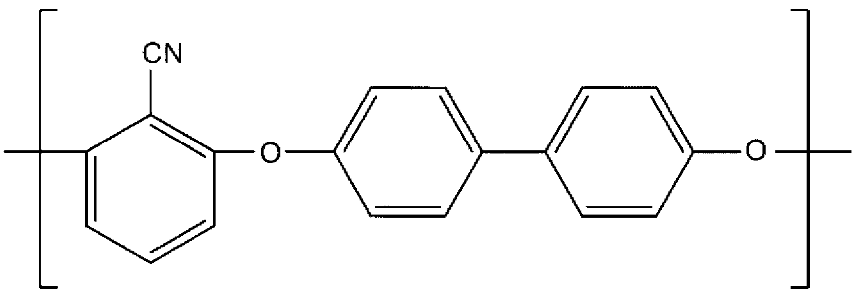

(1)

[Chem. 5]

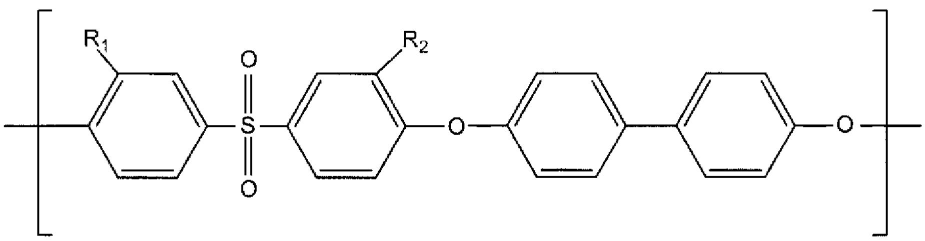

(2)

The component ratio (in mole) of the formula (1) is from 0.4 to 0.7; the component ratio (in mole) of the formula (2) is from 0.3 to 0.6; the sum of the component ratio (in mole) of the formula (1) and the component ratio (in mole) of the formula (2) is 1.00; $R_1$ and $R_2$ independently represent "$-SO_3M$" or "$-SO_3H$"; and M represents a metallic element.

The sulfonated polyarylene ether copolymer may be any of a random copolymer, a block copolymer, an alternating copolymer, and the like.

M (a metallic element) is not particularly limited, and examples thereof include sodium, potassium, and lithium.

In the sulfonated polyarylene ether copolymer, it is more preferable that the component ratio (in mole) of the formula (1) be from 0.4 to 0.7 and the component ratio (in mole) of the formula (2) be from 0.3 to 0.6, and it is more preferable that each of $R_1$ and $R_2$ in the formula (2) be "$-SO_3Na$" or "$-SO_3K$".

The sulfonated polyarylene ether copolymer is a material that does not change much during a long-term use of the membrane.

However, the sulfonated polyarylene ether copolymer tends to be degraded due to $\gamma$-ray sterilization, electron beam sterilization, or high-pressure steam sterilization. The semipermeable membrane according to the present embodiment is sterilized by EOG sterilization and thereby degradation of the sulfonated polyarylene ether copolymer is inhibited.

The sulfonated polyarylene ether copolymer may be obtained by a conventionally known method and the like. For

instance, 2,6-dichlorobenzonitrile, 3,3'-disulfo-4,4'-dichlorodiphenyl sulfone metal salt, and 4,4'-biphenol may be subjected to reaction in the presence of a basic compound for polymerization via an aromatic nucleophilic substitution reaction, and thereby a copolymer consisting of a hydrophobic segment represented by the formula (1) and a hydrophilic segment represented by the formula (2) may be obtained.

The polymerization may be caused at a temperature within the range of 0 to 350°C, and, preferably, the temperature is from 50 to 250°C. At below 0°C, the reaction tends not to proceed sufficiently, and at above 350°C, polymer degradation tends to begin.

The reaction may be caused in the absence of solvent, but preferably caused in solvent. Examples of the solvent that can be used include N-methyl-2-pyrrolidone, N,N-dimethylacetamide, N,N-dimethylformamide, dimethyl sulfoxide, diphenyl sulfone, and sulfolane; however, these examples are not restrictive and any solvent that is stable in an aromatic nucleophilic substitution reaction may be used.

Examples of the basic compound include sodium hydroxide, potassium hydroxide, sodium carbonate, potassium carbonate, sodium hydrogen carbonate, potassium hydrogen carbonate, and the like; however, these examples are not restrictive and those that can transform an aromatic diol into an active phenoxide structure may be used.

**In** an aromatic nucleophilic substitution reaction, water can be produced as a byproduct. **In** that case, toluene and/or the like may be made to coexist in the reaction system in addition to and independently from the polymerization solvent to remove water as an azeotrope, out of the system. As the method for removing water out of the system, use of a water-absorbing material such as molecular sieves may also be adopted.

**In** the case when the aromatic nucleophilic substitution reaction is caused in a solvent, it is preferable that the monomers be added in such a manner that a resulting polymer concentration from 5 to 50 mass% is to be achieved. When it is below 5 mass%, the degree of polymerization tends not to increase. When it is above 50 mass%, the viscosity of the reaction system tends to become too high to perform posttreatment of the reaction product with ease.

After the completion of the polymerization reaction, the solvent is removed from the reaction solution by evaporation, followed by rinsing the remaining product as needed, to obtain a desired polymer (a sulfonated polyarylene ether copolymer). Alternatively, the reaction solution may be added to a solvent that does not dissolve the polymer very well, to make the polymer precipitated as solid, followed by collection of the precipitate by filtration, and thereby the polymer may be obtained.

(Polyvinylpyrrolidone)

The polyvinylpyrrolidone is a polymer of monomers including at least N-vinylpyrrolidone.

The polyvinylpyrrolidone preferably includes a structural unit represented by the following formula (4).

[Chem. 6]

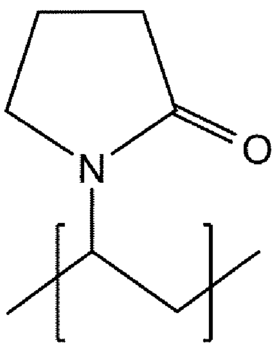

(4)

As the polyvinylpyrrolidone, products from BASF with weight average molecular weights of 9,000 (K17), 450,000 (K60), and/or 1,200,000 (K90) can be used, for example.

Preferably, the content ratio of (polyethersulfone)/(sulfonated polyarylene ether copolymer)/(polyvinylpyrrolidone) in the entirety of the semipermeable membrane is (75 to 90)/(7 to 22)/(3 to 18) (in mass%).

Preferably, the semipermeable membrane has a structure that is not uniform in a thickness direction (an asymmetric structure). More preferably, it has a structure that has a dense layer on the inner surface side thereof, with the pore size increasing continuously or discontinuously from the inner surface toward the outer surface (an asymmetric structure) (Fig. 5).

Examples of the semipermeable membrane having an asymmetric structure (an asymmetric membrane) include a membrane in which the above-mentioned dense layer serves as a separation-activating layer that substantially determines the pore size of the semipermeable membrane. It is impossible to directly measure the pore size of the

dense layer, and the molecular weight cut-off of SC dextran measured with the use of a semipermeable membrane having a dense layer is defined as the pore size of the dense layer. In the present invention, the molecular weight cut-off of SC dextran measured in this manner is preferably 100 kDa or less. From the viewpoint of durability of the membrane (filtration stability), the semipermeable membrane is preferably an asymmetric membrane.

In a hollow fiber membrane having an asymmetric structure, the aperture ratio of the inner surface is preferably different from the aperture ratio of the outer surface. The aperture ratio of the outer surface of the hollow fiber membrane is preferably 29% or more, more preferably 30% or more. The aperture ratio of the outer surface of the hollow fiber membrane is determined in an image that is taken with a scanning electron microscope (SEM).

Examples of the semipermeable membrane include semipermeable membranes called nanofiltration membranes (NF membranes), ultrafiltration membranes (UF membranes), and microfiltration membranes (MF membranes). The semipermeable membrane is preferably an ultrafiltration membrane and/or a microfiltration membrane.

Usually, the pore size of an NF membrane is from about 1 to 2 nm, the pore size of an UF membrane is from about 2 to 100 nm, and the pore size of an MF membrane is about 0.1 μm or more.

The shape of the semipermeable membrane is not particularly limited. The semipermeable membrane may be a flat membrane or a hollow fiber membrane (a hollow-fiber-type semipermeable membrane), and is preferably a hollow fiber membrane. A hollow fiber membrane provides an increased membrane area per module as compared to a flat membrane, which is an advantage.

<Semipermeable Membrane Module>

The present invention also relates to a semipermeable membrane module (a membrane separation instrument, a membrane separation apparatus) that comprises the above-mentioned semipermeable membrane and that is sterilized with ethylene oxide gas. The semipermeable membrane module is not particularly limited as long as it is a separation instrument (a separation apparatus) capable of performing separation (such as solid-liquid separation and liquid-liquid separation) with the use of the above-mentioned semipermeable membrane.

In a semipermeable membrane module (for example, a continuous hemofiltration instrument described above) having the above-mentioned hollow fiber membrane, it is preferable that the dialysate be made to flow on the outer side of the hollow fiber membrane and blood be made to flow on the inner side (inside the hollow portion) of the hollow fiber membrane (see Fig. 1).

<Production Method>

A method for producing the semipermeable membrane or the semipermeable membrane module comprising the semipermeable membrane comprises a step (a sterilization step) to sterilize the semipermeable membrane or the semipermeable membrane module comprising the semipermeable membrane with ethylene oxide gas.

(Sterilization Step)

In the sterilization step, the semipermeable membrane or the semipermeable membrane module comprising the semipermeable membrane is sterilized with ethylene oxide gas (EOG).

For instance, the semipermeable membrane module comprising the semipermeable membrane is subjected to sterilization treatment with ethylene oxide gas, in a state that it is placed inside a first package (a package for sterilization) that allows permeation of ethylene oxide gas (EOG) therethrough (namely, in a state that it is packaged inside the first package).

The first package is not particularly limited as long as it is a package that allows permeation of EOG therethrough (namely, that allows flow of EOG from inside to outside the package, and vice versa).

The package is not particularly limited as long as it is a vessel that is capable of accommodating the semipermeable membrane module and the like inside the vessel. From the viewpoint of reducing the entire volume of a commercial product which is the semipermeable membrane module packaged inside the package, the package is preferably a pouch that is capable of accommodating the semipermeable membrane module, for example.

For visibility inside the package, the package is preferably made of a transparent or translucent material. As the material of the package, a resin material may be used, for example.

The first package may be a package (such as a pouch) made of a combination of a gas-impermeable sheet that does not allow permeation of gas (including EOG) therethrough and a gas-permeable sheet that allows partial permeation of gas (including EOG) therethrough, for example. In this case, through the gas-permeable sheet, EOG can flow from inside to outside the package, and vice versa.

The gas-impermeable sheet may be, for example, a laminated sheet composed of a polyethylene terephthalate (PET) sheet (an inner layer) and a polyethylene sheet (an outer layer). The gas-permeable sheet may be, for example, a

sheet made of nonwoven fabric. The first package is not limited to the above ones, and it may be a package made of a gas-impermeable sheet.

The gas (EOG) used in EOG sterilization is not particularly limited as long as it is a gas containing ethylene oxide (EO). The EOG may be, for example, "Capox-20" (manufactured by NIPPON EKITAN Corporation) which is a mixed gas of EO (20 mass%) and $CO_2$ (80 mass%).

An example of the conditions for EOG sterilization is presented below.

Prevacuum pressure: -93 kPa
Exposure temperature: 43 to 53°C
Exposure humidity: 45 to 65%RH
Pressure during exposure: 80 to 120 kPa
Exposure duration: 360 minutes

(Other Steps)

After the sterilization step, the semipermeable membrane module accommodated inside the first package maintained in the sterilization state is placed in a second package (packaged inside the second package).

The second package is not particularly limited as long as it is a package capable of maintaining the sterilization state inside the package. Like the first package, the second package is also preferably a pouch.

The second package is made of a gas-impermeable sheet, for example. The gas-impermeable sheet to be used for the second package may be a laminated sheet composed of a resin sheet and a metal sheet, for example. An example of the laminated sheet is a laminated sheet composed of a nylon sheet (an inner layer), an aluminum sheet (an intermediate layer), and a low-density polyethylene layer (an outer layer).

Preferably, prior to packaging into the second package, a treatment (EOG removal treatment) is carried out to remove remaining EOG from the post-sterilization semipermeable membrane module (packaged inside the first package), by evaporation/diffusion and/or the like.

The EOG removal treatment removes EOG remaining in the semipermeable membrane module by, for example, placing the semipermeable membrane module (packaged inside the first package) in a reduced-pressure atmosphere (-93 kPa) to let EOG evaporate and diffuse and then performing aeration with sterilized air at a temperature from 1 to 30°C for one day to replace EOG with the sterilized air.

[Examples]

In the following, a more detailed description will be given of the present invention by way of Examples, but these Examples are not intended to limit the scope of the present invention.

(Preparation of Sulfonated Polyarylene Ether Copolymer)

3,3'-disulfo-4,4'-dichlorodiphenyl sulfone disodium salt, 2,6-dichlorobenzonitrile, 4,4'-biphenol, potassium carbonate, and molecular sieves were weighed into a four-necked flask, through which nitrogen was passed. NMP was added thereto and the resultant was stirred at 150°C for 50 minutes, followed by increasing the reaction temperature to the range of 195°C to 200°C to continue reaction until the viscosity of the system rose sufficiently. The resultant was left to cool, and after cooling, the sedimented molecular sieves were removed and the polymer was precipitated in water. The polymer thus obtained was rinsed in boiling water for 1 hour, followed by careful rinsing in pure water to completely remove the remaining potassium carbonate. Subsequently, the polymer from which potassium carbonate had been removed was dried, and thereby a sulfonated polyarylene ether copolymer (which may be abbreviated as "SPN" hereinafter) was obtained.

(Preparation of Hollow Fiber Membrane)

16.0 mass% of polyethersulfone (PES, manufactured by Sumitomo Chemical), 4.0 mass% of sulfonated polyarylene ether copolymer (SPN obtained in the above-described manner), 3.0 mass% of polyvinylpyrrolidone K-90 (PVP, manufactured by Nippon Shokubai), 46.2 mass% of N-methyl-2-pyrrolidone (NMP, manufactured by Mitsubishi Chemical), and 30.8 mass% of triethylene glycol (TEG, manufactured by Mitsubishi Chemical) were heated and dissolved into uniformity. The spinning dope thus obtained, together with a hollow-forming material (NMP/TEG/water=12/8/80), was discharged through a tube-in-orifice nozzle that was warmed to 55°C, passed through a 200-mm dry section that was shut off from the outside air by a spinning tube, coagulated in a coagulation bath (NMP/TEG/water=24/16/60) at 74°C, rinsed in a water bath at 80°C, and wound on a skeiner at a spinning rate of 37 m/min. The hollow fiber membranes (a bundle) thus obtained were rinsed with warm water at 70°C for removal of excess solvent and the like, and then immersed in 60-mass%

glycerol solution at 60°C so that the pores were filled with the aqueous glycerol solution. Centrifugation for liquid removal was performed to remove excess aqueous glycerol solution adhered to the surface of the hollow fiber membranes, followed by drying at 60°C.

Each hollow fiber membrane thus obtained had a dense layer on the inner surface side thereof, an inner diameter of 0.240 mm, and an outer diameter of 0.350 mm.

The SPN used here is a copolymer of the formula (1) and the formula (2), with the component molar ratio of (1) being 0.56 and the component molar ratio of (2) being 0.44.

(Preparation of Semipermeable Membrane Module)

The hollow fiber membrane bundle obtained in the above-described manner was placed inside a tubular vessel equipped with two nozzles respectively for liquid introduction and liquid discharge, and from both ends, urethane resin was injected. Subsequently, the cured urethane resin parts were cut off so as to open the ends of the hollow fiber membranes at the end faces, and thereby a semipermeable membrane module was obtained which had a membrane area (in terms of the inner diameter) of 1.7 $m^2$. Then, to each end of the semipermeable membrane module, a header cap equipped with a nozzle for blood introduction (or for blood discharge) was attached.

(EOG Sterilization, Etc.)

In the same manner as in the example provided above in the Embodiment section, sterilization treatment with ethylene oxide gas (EOG) was carried out in a state where the semipermeable membrane module comprising the semipermeable membranes was accommodated inside a first package (a package for sterilization).

The first package was a pouch made of a combination of a gas-impermeable sheet (a laminated sheet composed of a PET#12 sheet (an inner layer) and a polyethylene (PE40) sheet (an outer layer)) and a sheet allowing partial permeation of gas (a nonwoven fabric sheet, TYVEK1073B).

Conditions of the EOG sterilization are as described below.

EOG: Capox-20
Prevacuum pressure: -93 kPa
Exposure temperature: 43 to 53°C
Exposure humidity: 45 to 65%RH
Pressure during exposure: 80 to 120 kPa
Exposure duration: 360 minutes

After the sterilization step, the semipermeable membrane module accommodated inside the first package maintained in the sterilization state was subjected to EOG removal treatment, and placed inside a second package.

<Evaluation Test>

(Evaluation of Eluate from Each Sterilization Method)

A sample, which was made of the above-described hollow fiber membranes after EOG sterilization, was immersed in pure water at 70°C for 1 hour, and thereby an extracted liquid was obtained. The amount of pure water used for 1 g of the hollow fiber membranes was 100 mL.

For comparison purposes, a pre-EOG-sterilization hollow fiber membrane sample (non-sterilized) as well as pre-EOG-sterilization samples after γ-ray sterilization, electron beam sterilization, and high-pressure steam sterilization, respectively, were prepared, and extracted liquids were obtained in the same manner as described above.

Each of the extracted liquids was subjected to measurement of appearance, bubble disappearance, and UV absorbance.

Measurement of appearance, bubble disappearance, and UV absorbance was carried out in conformity with "(1) Appearance", "(2) Bubbles", and "(6) Ultraviolet absorption spectrum" of "V. Dialyzer quality and test method, 3. Elution test for dialysis membrane" included in "Standards for approval of dialysis-type artificial kidney devices" (Yakuhatsu No. 494, June 20, Showa 58 (1983), Notification from Director General of Pharmaceutical Affairs Bureau, the Ministry of Health and Welfare to all prefectural governors), in the manner described below.

[Appearance]

The test liquid (the extracted liquid) was rated as Passed when it was almost colorless and transparent and no

foreign matter was visible by the naked eye.

[Disappearance of Bubbles]

The test liquid was rated as Passed when almost all bubbles disappeared in 3 minutes after 5 mL of the test liquid in a stoppered test tube (with an inner diameter of about 15 mm and a length of about 200 mm) was vigorously shaken for 3 minutes.

[UV absorbance]

The absorbance of the test liquid and a blank test liquid (as a control) was measured at a path length of 10 mm and a wavelength of 220 to 350 nm. The test liquid was rated as Passed when the absorbance was 0.1 or less.
Results of measurement (evaluation) of appearance, bubble disappearance, and UV absorbance are shown in Table 1.

[Table 1]

| Evaluation items | Target | Non-sterilized | γ-Ray sterilization (25 kGy) | Electron beam sterilization | High-pressure steam sterilization | EOG sterilization |
|---|---|---|---|---|---|---|
| Appearance | Colorless and transparent | Passed | Passed | Passed | Passed | Passed |
| Bubble disappearance | In 3 min. | Passed | Passed | Passed | Passed | Passed |
| UV Absorbance (Evaluation) | ≤0.1 | 0.03 (Passed) | 0.9 | 0.3 | 1.2 | 0.02 (Passed) |

Referring to Table 1, between EOG sterilization and other sterilizations (γ-ray sterilization, electron beam sterilization, and high-pressure steam sterilization), no difference was observed in appearance and bubble disappearance.
However, in other sterilizations except EOG sterilization, the UV absorbance rose and did not satisfy the standard. Probably, this was caused by degradation of SPN (sulfonated polyarylene ether copolymer; relatively susceptible to degradation) of the hollow fiber membranes due to γ-ray sterilization, electron beam sterilization, or high-pressure steam sterilization.
In contrast, a rise of UV absorbance was not observed for the hollow fiber membranes after EOG sterilization. This indicates that the hollow fiber membranes after EOG sterilization did not produce SPN degradation products and had excellent quality.

(Molecular Weight Analysis (GPC) After γ-Ray Sterilization)

For the four types of samples shown in Table 2, chromatograms were obtained by GPC analysis. The chromatograms thus obtained are shown in Fig. 2. It should be noted that the hollow fiber membranes here are the above-mentioned hollow fiber membranes after EOG sterilization.
Conditions of the GPC measurement are as described below.

Apparatus: TOSOH HLC-8420GPC
Column: TSKgel SuperAWH-H×2
Solvent: DMAc/LiBr 30 mM
Flow speed: 0.3 mL/min
Concentration: Set at 0.005% to 0.2%
Amount injected: 10 μL
Temperature: 40°C
Detector: UV (269 nm), RI

[Table 2]

| | Mw | Mn | Mw/Mn | γ Ray dose | Analyzed subject |
|---|---|---|---|---|---|
| SPN polymer blank | 123900 | 60400 | 2.05 | (No irradiation) | Polymer alone |
| Hollow fiber membrane extracted liquid (50 kGy) | 31400 | 12200 | 2.57 | 50 kGy | Extracted liquid |
| Hollow fiber membrane extracted liquid (25 kGy) | 34600 | 17400 | 1.99 | 25 kGy | Extracted liquid |
| SPN polymer WET (50 kGy) | 32200 | 13400 | 2.41 | 50 kGy | Extracted liquid |

The results shown in Fig. 2 indicate that γ-ray sterilization reduced the size of the molecules of the sulfonated polyarylene ether copolymer (SPN).

(Relationship between γ-Ray Dose for Sterilization, Amount of SPN Elution, and Absorbance)

In Fig. 3, the relationship between the amount of elution of degraded SPN and the UV absorbance of the extracted liquid is shown. Referring to Fig. 3, the absorbance of the extracted liquid is proportional to the amount of SPN elution.
In Fig. 4, the relationship between γ-ray dose for sterilization and the amount of elution of degraded SPN is shown. Referring to Fig. 4, the amount of elution of degraded SPN is proportional to γ-ray dose for sterilization (the dose of γ ray applied to SPN). It is conceivable that degraded SPN tends to be produced in WET sterilization (sterilization of SPN by γ-ray irradiation in water) as compared to DRY sterilization (sterilization of SPN by γ-ray irradiation in a dry state).

(Storage Stability Test)

The above-described semipermeable membrane module was subjected to a stability test (a non-accelerated test and an accelerated test shown in Table 3), and then, with the use of the hollow fiber membranes of the semipermeable membrane module after the test, extracted liquid was obtained in the same manner as above. This extracted liquid was subjected to measurement of oxidation indices, namely, KI (potassium iodide), $H_2O_2$ (hydrogen peroxide), and ΔpH (the amount of change in pH before and after the test).

[Table 3]

| | Non-accelerated test | Accelerated test (Conditions: 60°C, 1 week) | |
|---|---|---|---|
| | | First package (Pouch for EOG sterilization) | First package + Second package (Aluminum pouch) |
| KI | 0.00 | 0.08 | 0.00 |
| $H_20_2$ | 1.7 | 28.9 | 0.6 |
| ΔpH | 0.2 | 0.1 | 0.2 |

The results shown in Table 3 indicate that oxidation of the hollow fiber membranes proceeded when the hollow fiber membranes were placed inside the first package alone because the first package (a pouch for EOG sterilization) allowed permeation of oxygen. This indicates that packaging in both the first package and the second package makes it possible to inhibit degradation of the semipermeable membrane module.

(Proinflammatory Mediator Adsorption Rate)

To 1000 mL of porcine whole blood containing heparin added thereto, 24 μg of each of IL-6 as a negatively charged substance and IL-8 as a positively charged substance was added to prepare a sample blood.
In the semipermeable membrane module (a continuous hemofiltration instrument) (before and after EOG sterilization treatment), the sample blood was circulated for 1 hour under conditions of a blood flow rate (Qb) of 100 mL/minute and a filtrate flow rate (Qf) of 10 mL/minute.
After circulation, the entire amount of the sample blood was collected, and the amounts of IL-6 and IL-8 in the sample blood were measured by enzyme-linked immunosorbent assay (ELISA). From the measured values and the amounts added to the sample blood, decrements of IL-6 and IL-8 were calculated, which were regarded as the adsorption rates of IL-6 and IL-8, respectively. The IL-6 and IL-8 adsorption rates thus obtained are shown in Table 4.

(Platelet Remaining Rate)

In the semipermeable membrane module (a continuous hemofiltration instrument) (before and after EOG sterilization treatment), 1000 mL of porcine whole blood containing heparin added thereto was circulated for 1 hour under conditions of a blood flow rate (Qb) of 100 mL/minute and a filtrate flow rate (Qf) of 10 mL/minute. After circulation, the entire amount of the sample blood was collected. With the use of a blood cell counter, platelets in the blood after 1-hour circulation and those in the blood before circulation were measured. The resulting measured values were used for calculation of (platelet remaining rate)=(blood after 1-hour circulation)/(blood before circulation). Results of measurement of the platelet remaining rate are shown in Table 4.

[Table 4]

| | Proinflammatory mediator adsorption rate (%) | | Platelet Remaining Rate (%) |
|---|---|---|---|
| | IL-6 | IL-8 | |
| Before EOG sterilization | 91 | 94 | 83 |
| After EOG sterilization | 91 | 92 | 100 |

Referring to Table 4, the adsorption rate of negatively charged substance interleukin 6 (IL-6) and that of positively charged substance interleukin 8 (IL-8) after EOG sterilization were 91% and 92%, respectively, indicating that no degradation of performance (no influence of sterilization treatment) was observed.

(Filtration Stability: ΔTMP)

In the module (before and after EOG sterilization treatment), bovine blood which contained citric acid added thereto for coagulation inhibition and which had been adjusted to a hematocrit content of 30±2% and a protein concentration of 6 to 7 g/dL was circulated for 150 minutes under conditions of a blood flow rate (Qb) of 400 mL/minute and a filtration flow rate (Qf) of 100 mL/minute. A value of TMP (the measured value after a lapse of a specific time period up to 150 minutes) from which TMP after a lapse of 15 minutes was subtracted was defined as ΔTMP. Results of the measurement of ΔTMP after a lapse of 150 minutes are shown in Table 5.

(Performance in Aqueous System)

The module (before and after EOG sterilization treatment) was subjected to the below-described measurement of performance (performance in aqueous system) (1) to (3). Results of the measurement are shown in Table 5.

(1) (Measurement of Ultrafiltration Coefficient UFR of Pure Water)

The blood outlet circuit of the semipermeable membrane module was sealed for dead-end filtration. After a pressure tank was filled with pure water maintained at 37°C, the pressure was regulated with a regulator to feed the pure water into the hollow fiber membranes of the semipermeable membrane module placed inside a thermostatic chamber maintained at 37°C, and the amount of filtrate flowing out to the outside of the hollow fiber membranes was measured with a measuring cylinder. The transmembrane pressure (TMP) was set at TMP=(Pi+Po)/2. Pi is the pressure at the module inlet, and Po is the pressure at the module outlet. TMP was changed and the filtration flow rate was measured at four positions; from the slope of the straight line thus obtained, the permeability UFR (mL/hr/$m^2$/mmHg) of the module was calculated. At this point, the correlation coefficient between TMP and the filtration flow rate needs to be 0.999 or more. For reducing the pressure loss error attributable to the circuit, measurement was carried out at a TMP within the range of 100 mmHg or less.

(2) (Measurement of Clearance (CL))

According to "The method of hemodialyzer performance assessment, 2012", clearance (CL) of urea (UN), vitamin B12 (Vb), and myoglobin (myo) was measured. Clearance (CL) and mass balance error (MBE) thereof are shown in Table 5. Clearance (CL) is an index of substance removal, and mass balance error (MBE) is an index of measurement errors.

Physiological saline in which 0.1% urea (manufactured by Nacalai Tesque), 0.002% vitamin B12 (manufactured by Nacalai Tesque), and 0.01% myoglobin (manufactured by Kishida Chemical) were dissolved was made to flow single-pass at a flow rate (QBin) of 200 mL/min, without being filtered, through the blood channel side of the semipermeable membrane module which had been primed and wetted with physiological saline, and through the dialysate-side channel,

the dialysate was made to flow at a flow rate (Qd) of 500 mL/min. The solute concentration of the measurement liquid at the inlet of the hollow fiber membranes, the solute concentration of the measurement liquid at the outlet, and the amount of liquid supply were measured to calculate clearance (CL).

Clearance (CL) is represented by the following equation.

CL (mL/min)=(((inlet concentration)-(outlet concentration))/(inlet concentration))×(amount of liquid supply)

Mass balance error (MBE) is represented by the following equation.

$$MBE=(MB-MD)/MB$$

*MB=(blood-side inlet flow rate)×((inlet concentration)-(outlet concentration)), MD=(dialysate flow rate)×(dialysate concentration)

(3) (Calculation of Protein Leak Amount (Total Protein Loss: TPL))

Bovine plasma with added citric acid was adjusted to a protein concentration of 6 to 7 g/dL and fed to the semipermeable membrane module at 37°C at 200 mL/min for plasma filtration at a filtration flow rate of 15 mL/min. The filtrate was returned back to the plasma for circulation. The filtration flow rate was measured every 15 minutes, and the filtrate was collected from the semipermeable membrane module. The concentration of protein contained in the filtrate was measured. Measurement of the protein concentration of the plasma was carried out with the use of an in vitro diagnostic kit (MicroTP-Test Wako, manufactured by Wako Pure Chemical Industries). The average protein leak amount was determined based on the data taken over a lapse of 2 hours, and the protein leak amount (TPL) in terms of 3-L fluid removal was calculated, which was subjected to evaluation. TPL decrement in blood is desirably low, and therefore TPL leak into the dialysate is preferably low (that is, TPL concentration in the dialysate is preferably low).

[Table 5]

| | Filtration stability ΔTMP (mmHg) | Performance in aqueous system | | | | TPL (g/3L) |
|---|---|---|---|---|---|---|
| | | UFR ($mL/hr/m^2/mmHg$) | CL ($mL/min/m^2$) [MBE (%)] | | | |
| | | | CLun | CLvb | CLmyo | |
| Before EOG sterilization | 12 | 84 | 168 [-3.3] | 94 [6.4] | 33 [20] | 0.0 |
| After EOG sterilization | 11 | 80 | 170 [0.8] | 89 [-4.4] | 33 [23] | 0.0 |

The results shown in Table 5 indicate that ΔTMP before and after EOG sterilization treatment is small, suggesting that the semipermeable membrane (module) according to the present invention has excellent filtration stability.

It should be noted that the ΔTMP value measured after a lapse of 150 minutes is preferably 15 mmHg or less.

Moreover, the semipermeable membrane (module) according to the present invention after EOG sterilization maintained high UFR and high clearance and exhibited low TPL leak amount, suggesting that it has suitable properties for use in CRRT and the like.

## Claims

**1.** A semipermeable membrane comprising:

a sulfonated polyarylene ether copolymer, wherein
the sulfonated polyarylene ether copolymer includes a hydrophobic segment repeating unit represented by a formula (1) below and a hydrophilic segment repeating unit represented by a formula (2) below as copolymerization components:

[Chem. 1]

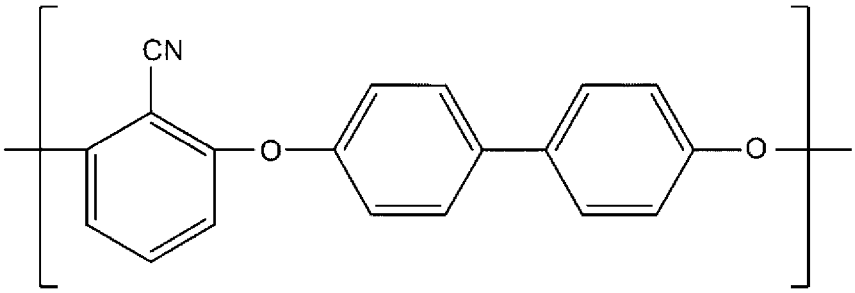

(1)

[Chem. 2]

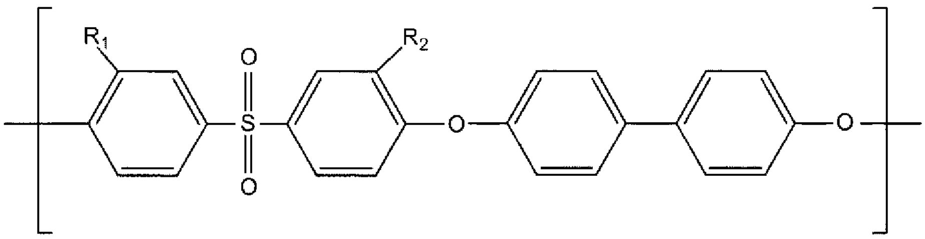

(2)

where a component ratio (in mole) of the formula (1) is from 0.4 to 0.7, a component ratio (in mole) of the formula (2) is from 0.3 to 0.6, a sum of the component ratio (in mole) of the formula (1) and the component ratio (in mole) of the formula (2) is 1.00, each of $R_1$ and $R_2$ represents "$-SO_3M$", and M represents a metallic element, and the semipermeable membrane is sterilized with ethylene oxide gas.

2. The semipermeable membrane according to claim 1, comprising polyethersulfone, the sulfonated polyarylene ether copolymer, and polyvinylpyrrolidone.

3. The semipermeable membrane according to claim 1 or 2, for continuous renal replacement therapy.

4. The semipermeable membrane according to any one of claims 1 to 3, having a structure that is not uniform in a thickness direction.

5. A semipermeable membrane module comprising the semipermeable membrane according to any one of claims 1 to 4, wherein

the semipermeable membrane module is sterilized with ethylene oxide gas.

6. The semipermeable membrane module according to claim 5, for continuous renal replacement therapy.

FIG.1

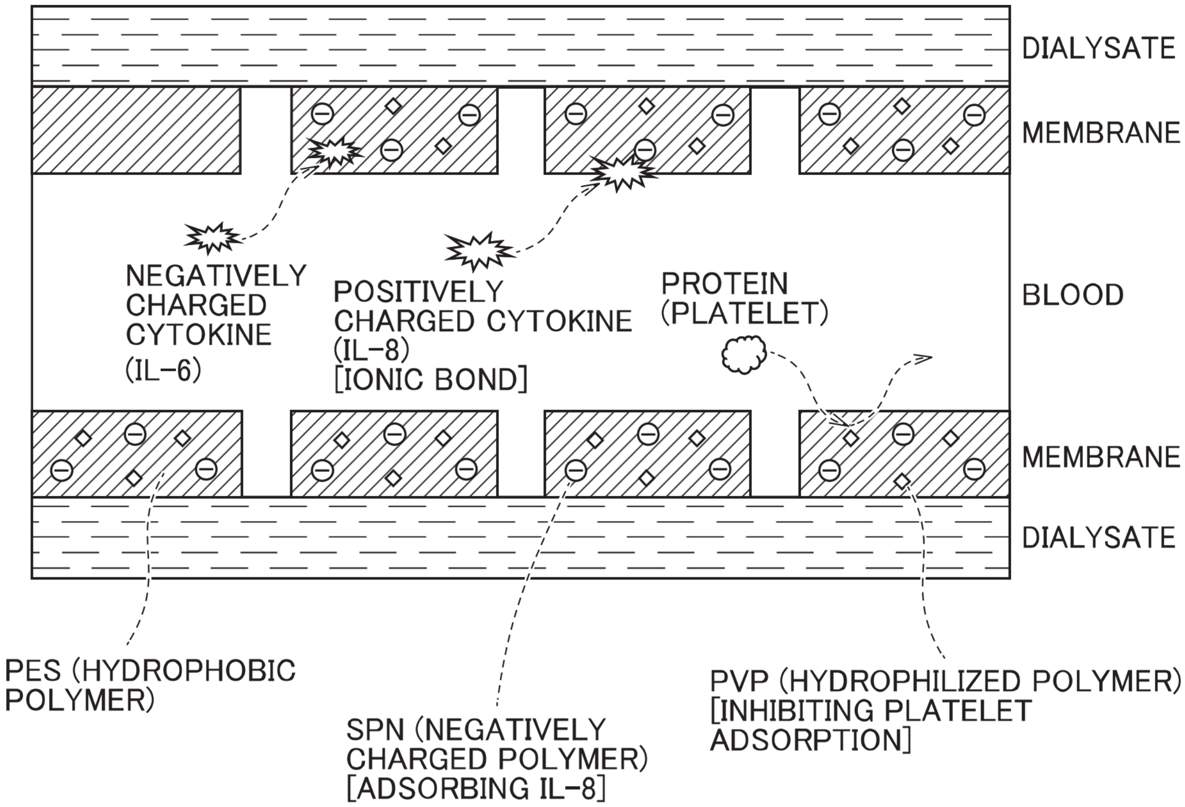
DIALYSATE
MEMBRANE
NEGATIVELY CHARGED CYTOKINE (IL-6)
POSITIVELY CHARGED CYTOKINE (IL-8) [IONIC BOND]
PROTEIN (PLATELET)
BLOOD
MEMBRANE
DIALYSATE
PES (HYDROPHOBIC POLYMER)
SPN (NEGATIVELY CHARGED POLYMER) [ADSORBING IL-8]
PVP (HYDROPHILIZED POLYMER) [INHIBITING PLATELET ADSORPTION]

FIG.2

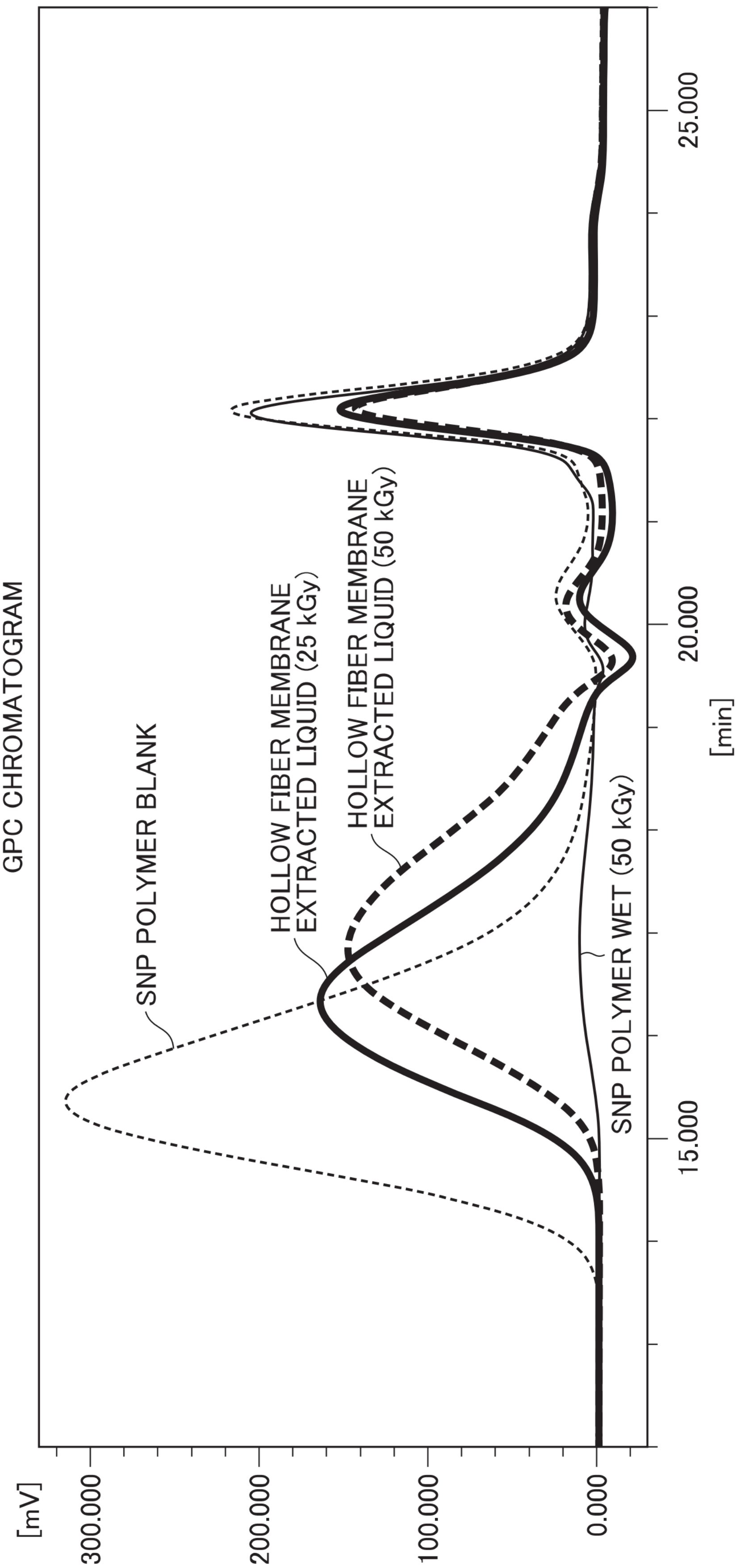
GPC CHROMATOGRAM
[mV]
300.000
200.000
100.000
0.000
SNP POLYMER BLANK
HOLLOW FIBER MEMBRANE
EXTRACTED LIQUID (25 kGy)
HOLLOW FIBER MEMBRANE
EXTRACTED LIQUID (50 kGy)
SNP POLYMER WET (50 kGy)
15.000
20.000
25.000
[min]

FIG.3

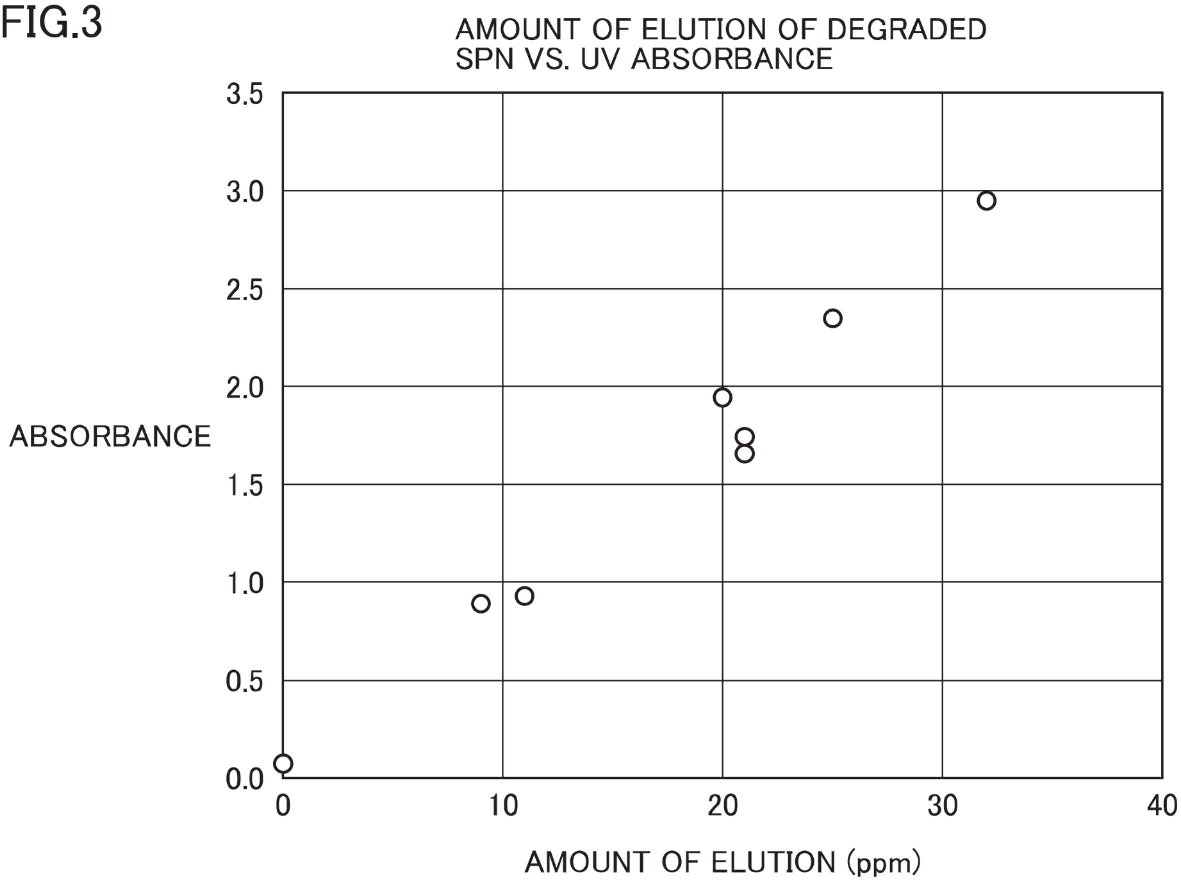
AMOUNT OF ELUTION OF DEGRADED
SPN VS. UV ABSORBANCE
ABSORBANCE
3.5
3.0
2.5
2.0
1.5
1.0
0.5
0.0
0
10
20
30
40
AMOUNT OF ELUTION (ppm)

FIG.4

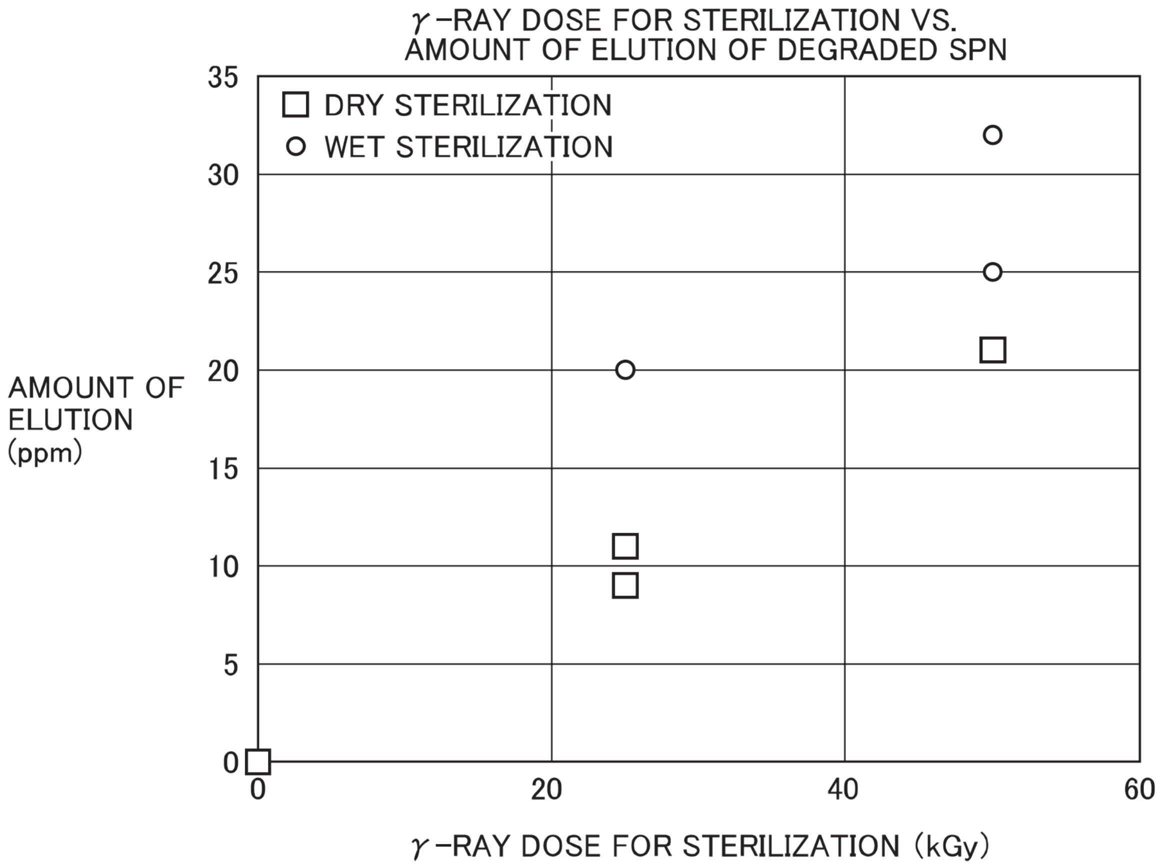
γ-RAY DOSE FOR STERILIZATION VS.
AMOUNT OF ELUTION OF DEGRADED SPN
DRY STERILIZATION
WET STERILIZATION
AMOUNT OF
ELUTION
(ppm)
35
30
25
20
15
10
5
0
0
20
40
60
γ-RAY DOSE FOR STERILIZATION (kGy)

FIG.5

| | EXAMPLE 1 |
|---|---|
| OUTER SURFACE |  |
| INNER SURFACE |  |
| CROSS SECTION | 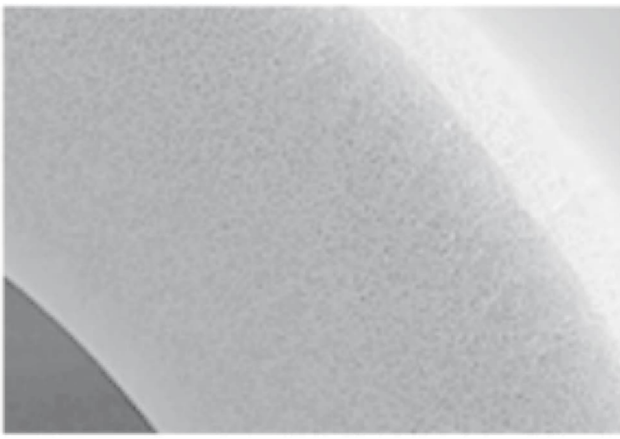 |

**INTERNATIONAL SEARCH REPORT**

International application No. **PCT/JP2024/000352**

**A. CLASSIFICATION OF SUBJECT MATTER**

***B01D 71/52***(2006.01)i; ***A61M 1/18***(2006.01)i; ***A61M 1/34***(2006.01)i; ***A61M 1/36***(2006.01)i; ***B01D 63/00***(2006.01)i; ***B01D 65/02***(2006.01)i; ***B01D 69/00***(2006.01)i; ***B01D 71/44***(2006.01)i; ***B01D 71/68***(2006.01)i; ***B01D 71/80***(2006.01)i; ***B01D 71/82***(2006.01)i

FI: B01D71/52; A61M1/18 500; A61M1/34 100; A61M1/36 165; B01D63/00; B01D65/02 500; B01D69/00; B01D71/44; B01D71/68; B01D71/80; B01D71/82 500

According to International Patent Classification (IPC) or to both national classification and IPC

**B. FIELDS SEARCHED**

Minimum documentation searched (classification system followed by classification symbols)

B01D53/22, 61/00-71/82, C02F1/44, A61M1/00-38, 60/00-90, C08G65/00-67/04, C08K3/00-13/08, C08L1/00-101/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2017/064936 A1 (TOYOBO CO., LTD.) 02 April 2017 (2017-04-02)<br>paragraphs [0021]-[0085] | 1, 4-5<br>2-3, 6 |
| Y<br>A | WO 2018/003949 A1 (TOYOBO CO., LTD.) 04 January 2018 (2018-01-04)<br>paragraphs [0071]-[0077] | 1, 4-5<br>2-3, 6 |
| Y<br>A | WO 2018/066631 A1 (TOYOBO CO., LTD.) 12 April 2018 (2018-04-12)<br>paragraphs [0054]-[0061] | 1, 4-5<br>2-3, 6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 March 2024** | **19 March 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
PCT/JP2024/000352

C. DOCUMENTS CONSIDERED TO BE RELEVANT

| Category* | Citation of document, with indication, where appropriate, of the relevant passages | Relevant to claim No. |
|---|---|---|
| Y<br>A | JP 2016-64122 A (TOYOBO CO., LTD.) 28 April 2016 (2016-04-28)<br>paragraphs [0002]-[0092] | 1, 3-6<br>2 |
| Y | JP 63-97202 A (TORAY INDUSTRIES, INC.) 27 April 1988 (1988-04-27)<br>page 4, lower right column, line 19 to page 5, upper left column, line 5 | 1, 3-6 |
| Y | WO 2017/104558 A1 (TOYOBO CO., LTD.) 22 June 2017 (2017-06-22)<br>paragraph [0028] | 1, 3-6 |

Form PCT/ISA/210 (second sheet) (July 2022)

**INTERNATIONAL SEARCH REPORT**
**Information on patent family members**

International application No.
**PCT/JP2024/000352**

| Patent document cited in search report | Publication date (day/month/year) | Patent family member(s) | Publication date (day/month/year) |
|---|---|---|---|
| WO 2017/064936 A1 | 02 April 2017 | US 2019/0076791 A1<br>paragraphs [0046]-[0116]<br>CN 108136344 A | |
| WO 2018/003949 A1 | 04 January 2018 | (Family: none) | |
| WO 2018/066631 A1 | 12 April 2018 | JP 2018-58040 A | |
| JP 2016-64122 A | 28 April 2016 | (Family: none) | |
| JP 63-97202 A | 27 April 1988 | (Family: none) | |
| WO 2017/104558 A1 | 22 June 2017 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6817240 B
- JP 2018171431 A
- JP 2001070767 A
- JP 2728814 B
- JP 5064662 A


### Non-patent literature cited in the description


- *The Journal of Artificial Organs*, 2014, vol. 43 (3), 233-237